# EUROPEAN PATENT APPLICATION

(11) **EP 4 529 844 A1**
(43) Date of publication of application: **02.04.2025**
(21) Application number: 23200546.2
(22) Date of filing: 28.09.2023
(51) Int. Cl.: A61B 6/00

(54) **X-RAY IMAGING**

(71) Applicant: Koninklijke Philips N.V., 5656 AG Eindhoven (NL)
(72) Inventor: KOEPNICK, Johannes, Eindhoven (NL); LUNDT, Bernd, Eindhoven (NL); MAY, Jan Marek, 5656AG Eindhoven (NL)
(74) Representative: Philips Intellectual Property & Standards

(57) **Abstract**

Proposed concepts thus aim to provide schemes, solutions, concept, designs, methods and systems pertaining to a method for assessing the set-up of a patient and a medical x-ray imaging device for examination of the patient. In particular, embodiments aim to provide a method for analysing patient position data from a sensor to determine the pose of the patient to be examined and determine a set-up assessment value based on the determined patient pose and at least one examination parameter of the x-ray device set-up.

## Description

### FIELD OF THE INVENTION

This invention relates to the field of x-ray imaging, and more specifically, to the field of the set-up of a medical x-ray imaging examination.

### BACKGROUND OF THE INVENTION

A patient and a medical x-ray device may be set-up for examination of the patient. Normally, an operator selects the desired examination parameters for the required examination on a user interface of the x-ray device before the patient is positioned in the field of view of the x-ray system. Automatic exposure control chambers (AEC) of the x-ray device are then automatically selected by the system based on the selected examination parameters. It is important that the AEC chambers are correctly selected and located to correspond to the position of the patient in order to ensure correct dose control. If the patient is positioned incorrectly for the selected exam, the image acquired by the x-ray device may be of reduced quality, or the examination may result in an overexposure leading to an unnecessary dose for the patient.

### SUMMARY OF THE INVENTION

The invention is defined by the claims.

According to examples in accordance with an aspect of the invention, there is provided a method for assessing the set-up of a patient and a medical x-ray imaging device for examination of the patent.

The method comprises: analysing patient position data from a position sensor to determine a pose of the patient to be examined, wherein the patient position data describes the position of the patient with respect to the x-ray imaging device; and determining a set-up assessment value based on the determined patient pose and at least one examination parameter of the x-ray device set-up.

Proposed concepts thus aim to provide schemes, solutions, concept, designs, methods and systems pertaining to a method for assessing the set-up of a patient and a medical x-ray imaging device for examination of the patient. In particular, embodiments aim to provide a method for analysing patient position data from a sensor to determine the pose of the patient to be examined and determine a set-up assessment value based on the determined patient pose and at least one examination parameter of the x-ray device set-up.

In particular, it is proposed that by using data from a position sensor to determine the position of the patient relative to the x-ray imaging device, the position of the patient can be assessed in relation to selected examination parameters of the x-ray device set-up. Therefore, the method provides a means for assessing whether the actual view position of the x-ray imaging device matches the required view position for the selected examination parameters. Hence, the disclosed invention provides a means for ensuring correct examination of the patient is carried out.

In the case where there is a mismatch between the set-up of the x-ray device and the position of the patient, the patient may receive an unnecessarily high exposure to x-rays if the AEC chambers are incorrectly selected. AEC chambers enable the automatic termination of x-ray exposure when a predetermined dose of radiation has been received. Therefore, if the incorrect AEC chambers are selected then the dose received by the patient may be higher than expected. Further, if the patient is positioned incorrectly, the image produced may be of reduced quality, or of the incorrect anatomy and therefore further examination of the patient may be required. This will result in further exposure of the patient. The method proposed herein may thus provide a mechanism for ensuring that the x-ray device set-up and the position of the patient are configured for the required examination of the patient, thereby ensuring that there is no unnecessary exposure of the patient to x-ray radiation.

Embodiments of the proposed method may provide a mechanism for checking that the operator has inputted the correct examination parameters, and that the patient is positioned correctly in front of the x-ray device. The present invention therefore has the advantage that it facilitates the determination of whether there is a mismatch between the selected examination parameters and the current position of the patient. Implementation of such a method may therefore result in a lower occurrence of incorrect or low-quality imaging of patients.

Ultimately, an improved method for assessing the set-up of a patient and a medical x-ray imaging device for examination of a patient may be provided by the proposed concept(s).

In some embodiments, determining a set-up assessment value based on the determined patient pose and the at least one examination parameter may comprise analysing information with respect to the view position of the x-ray imaging device, the identity of at least one anatomical feature of the patient, and the laterality of the at least one anatomical feature. In this way, the method provides a mechanism for analysing key pieces of information that generally define the type of examination to be carried out on the patient.

In some embodiments, determining a set-up assessment value may comprise determining from the pose of the patient expected examination parameters, comparing the expected examination parameters to the parameters of the x-ray device set-up. Optionally, the method may further comprise determining an alert signal based on the comparison of the expected examination parameters and the examination parameters of the x-ray device set-up. In this way, the position of the patient may be directly translated into values corresponding to the examination parameters of the x-ray device set-up selected by the operator. Further, the production of an alert signal enables the operator to be alerted if the patient is incorrectly positioned for the selected examination and hence provides the opportunity for the operator to make changes to the set-up to enable the correct examination to be carried out.

In some embodiments, analysing patient position data comprises identifying at least one anatomical feature of the patient, determining the orientation and position of the at least one anatomical feature, determining the laterality of the at least one anatomical feature, and determining the view position of the x-ray imaging device. In this way, information is determined from the patient position that corresponds to the information contained in examination parameters of the x-ray device set-up and defines the examination to be carried out.

In some embodiments, analysing patient position data employs at least one neural network. In this way, an efficient method of analysing the patient position data from the position sensor to determine the pose of the patient is provided.

In some embodiments, the position sensor may comprise at least one of, an RGB camera, an RGB-D camera, a laser position sensor, a radar position sensor, an accelerometer, a gyroscope, and an ultrasound device. In this way, appropriate patient position data can be obtained that allows for the determination of the position and orientation of the patient relative to the x-ray imaging device.

In some embodiments an examination parameter of the x-ray device set-up may comprise at least one of an anatomical feature of the patient to be examined, an orientation of an anatomical feature of the patient, a view position of the x-ray imaging device, and an automatic exposure control (AEC) chamber of the x-ray imaging device to be used. In this way, the examination parameters of the device may define a category of examination to be carried out.

In some embodiments, a set-up assessment value may comprise at least one of a value describing the suitability of the patient pose with respect to the examination parameters of the x-ray device set-up, and a target adjustment to be made to the set-up. In this way, the proposed method may provide the operator with information on the quality of the set-up and possible changes to be made to the set-up to correct it.

In some embodiments, a target adjustment to be made to the set-up may comprise at least one of an adjustment to the pose of the patient, an adjustment to the position of the x-ray imaging device, and an adjustment to the examination parameters of the x-ray device set-up. In this way, the method is provided with a mechanism for adjusting the set-up to ensure the patient pose corresponds to the selected examination parameters.

In addition, embodiments of the present invention provide concepts for a non-transitory computer readable medium comprising code stored thereon that, when executed, performs a method for assessing the set-up of a patient and a medical x-ray imaging device, the method comprising: analysing position data from a position sensor to determine a pose of the patient to be examined, wherein the patient position data describes the position of the patient with respect to the x-ray imaging device; and determining a set-up assessment value based on the determined patient pose and at least one examination parameter of the x-ray device set-up.

In another embodiment, a non-transitory computer readable medium is provided storing instructions for performing a method for assessing the set-up of a patient and a medical x-ray imaging device. When executed by at least one processor, the instructions cause the at least one processor to analyse patient position data from a position sensor to determine a pose of the patient to be examined, wherein the patient position data describes the position of the patient with respect to the x-ray imaging device. Further, the processor determines a set-up assessment value based on the determined patient pose and at least one examination parameter of the x-ray device set-up.

According to another aspect, there is provided a system for assessing the set-up of a patient and a medical x-ray imaging device for examination of the patient, comprising: an analysis component configured to analyse patient position data from a position sensor to determine a pose of the patient to be examined, wherein the patient position data describes the position of the patient with respect to the x-ray imaging device; and a processor configured to determine a set-up assessment value based on the determined patient pose and at least one examination parameter of the x-ray device set-up.

Thus, there may be proposed concepts for a method of assessing the set-up of a patient and a medical x-ray imaging device for examination of the patient, based on patient position data from a position sensor and at least one examination parameter of the x-ray device set-up.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for assessing the set-up of a patient and a medical x-ray imaging device for examination of the patient according to a proposed embodiment;
Fig. 2A is an illustration of a set-up of a patient and medical x-ray device for a lateral chest examination of the patient;
Fig. 2B is an illustration of a set-up of a patient and a medical x-ray device for a posterior-anterior (PA) chest examination of the patient;
Fig. 3 is an illustration of an RGB image of a patient on which key point identification has been used to determine a patient pose for a PA chest examination of a patient; and
Fig. 4 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to the assessment of a set-up of a patient and a medical x-ray imaging device for examination of the patient. According to proposed concepts, a number of possible solutions may be implemented separately or jointly. That is, although these possible solutions be described below separately, two or more of these possible solutions may be implemented in one combination or another.

Embodiments of the invention aim to provide a method for assessing a set-up of a patient and a medical x-ray imaging device for examination of the patient. This is achieved by analysing patient position data from a position sensor to determine the pose of the patient and then determining a set-up assessment value based on the determined patient pose and at least one examination parameter of the x-ray device set-up.

Proposed concepts thus aim to provide schemes, solutions, concepts, designs, methods, and systems pertaining to assessing the set-up of a patient and a medical x-ray imaging device for examination of the patient. In particular, embodiments aim to provide a method for assessing the set-up of a patient and a medical x-ray imaging device for examination of the patient, which includes analysing patient position data to determine a pose of the patient to be examined and determining a set-up assessment value based on the determined patient pose and an examination parameter of the x-ray device set-up.

It is proposed that by analysing the position of the patient the set-up of the patient and the x-ray imaging device can be assessed in relation to the selected examination parameters of the x-ray device set-up. For example, the proposed invention may provide a method of determining whether the patient is positioned suitably for the examination selected on the x-ray imaging device. In this way, the user may be alerted if the patient is positioned incorrectly for the desired examination.

Accurate positioning of the patient is required to ensure correct exposure control. The selected examination parameters on the x-ray device determine which AEC chambers are activated. These chambers ensure the termination of exposure once the radiation received by the chambers has reached a predetermined level, and thus enable careful patient dose control. If there is a mismatch between the patient's position and the selected examination parameters, the incorrect AEC chambers may be selected, and the patient may receive a higher dose than intended.

Implementation of the method disclosed herein, may result in reduced incidence of incorrect imaging of patients. Incorrect selection of examination parameters, or incorrect patient positioning may lead to the production of low-quality images, or the wrong examination being carried out. This may lead to the examination having to be repeated, resulting in further exposure for the patient. Thus, the present invention has the advantage of reducing the incidences in which repeat examinations of a patient are required.

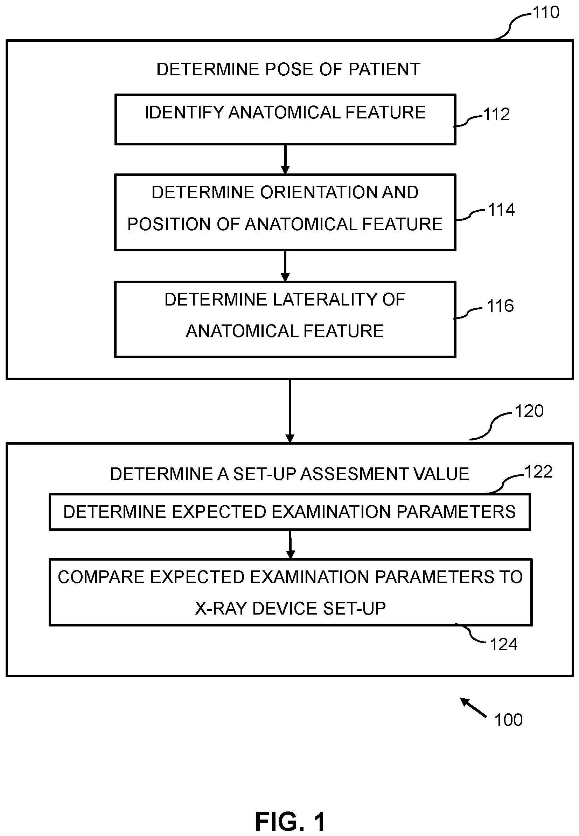

Referring now to Fig. 1 there is depicted a simplified flow diagram of a method 100 for assessing the set-up of a patient and a medical x-ray imaging device for examination of the patient according to a proposed embodiment.

The method starts with a step 110, comprising determining the pose of the patient to be examined. This involves analysing patient position data from a position sensor, wherein the patient position data describes the position of the patient with respect to the x-ray imaging device. In this exemplary method, the position sensor is at least one of an RGB camera, and RGB-D camera, a laser position sensor, a radar position sensor, an accelerometer, a gyroscope, and an ultrasound device. Analysing the patient position data, in this example, involves the use of at least one neural network which provides an efficient way of analysing the data. In this exemplary method, step 110 further comprises the three sub-steps 112, 114, and 116:
Step 112 - identify from the patient position data at least one anatomical feature of the patient to be examined. In this exemplary embodiment, this involves determining anatomical features of the patient that are in the field-of-view of the x-ray imaging device.
Step 114 - determine the orientation and position of at least one anatomical feature.
Step 116 - determine the laterality of at least one anatomical feature. The laterality discussed here will be understood by the skilled person in the context of a DICOM tag. Correspondingly the laterality of the anatomical feature may be at least one of: left, right, unpaired, and both left and right.

In this exemplary method, determining the patient pose involves comparing the identified orientation, position and laterality of different identified anatomical features.

The method then proceeds to the step 120, comprising determining a set-up assessment value based on the determined patient pose and at least one examination parameter of the x-ray device set up. In this exemplary embodiment, the step 120 comprises analysing information with respect to the view position of the x-ray device, the identity of at least one anatomical feature of the patient, and the laterality of the at least one anatomical feature. The set-up assessment value determined in step 120, in this example, comprises at least one of a value describing the suitability of the patient pose with respect to the examination parameters of the x-ray device set-up, and a target adjustment to be made to the set-up (e.g., an adjustment to the pose of the patient, an adjustment to the position of the x-ray imaging device, or an adjustment to the examination parameters of the x-ray device set-up).

In this exemplary method, step 120 comprises two sub-steps, 122 and 124: step 122 comprises determining from the pose of the patient, expected examination parameters; and step 124 comprises comparing the expected examination parameters to the examination parameters of the x-ray device set-up. An examination parameter, in this exemplary method, comprises at least one of, an anatomical feature of the patient to be examined, and orientation of an anatomical feature of the patient, a laterality of an anatomical feature of the patient, a view position of the x-ray imaging device, and an automatic exposure control chamber of the x-ray imaging device to be used. In this way, the method allows for the direct comparison of the patient pose and the selected examination parameters.

In the method outlined above, a set-up assessment value is determined. Optionally, this set-up assessment value may be used further to produce an alert signal to the operator in the case of a mismatch between the examination parameters of the x-ray device set-up and the determined pose of the patient. In this alternative example, the alert signal may be visual or auditory.

Similarly, other steps of the method detailed above may be implemented differently in alternative embodiments. For instance, in step 110 any suitable position sensor may be used to obtain data on the position of the patient relative to the x-ray imaging device and the disclosure is not limited to those given above. Further, the analysis of the patient position data is not required to be carried out by a neural network.

In summary, the proposed method provides a way of checking whether a patient has been positioned correctly in front of an x-ray imaging device to correspond to selected examination parameters of the x-ray device set-up. Conversely, the method may be used to check that the correct examination parameters have been selected to correspond to the position of the patient in front of the x-ray device. Incorrect set-up of a patient and an x-ray imaging device may result in a low-quality image, or an unexpectedly high dosage to the patient. Thus, the present invention provides a mechanism for assessing the set-up of a patient and a medical x-ray imaging device to ensure correct configuration for the required examination. This is achieved by analysing data from a suitable position sensor to determine the pose of the patient before the examination is carried out. This is then compared to the selected examination parameters of the x-ray device set-up to produce a set-up assessment value, which in some embodiments may either quantify the suitability of the patient pose for the selected examination or provide details of an adjustment to be made to the set-up to ensure the correct examination is carried out.

Typically, medical x-ray images are categorised using DICOM tags. These metadata tags are digitally combined with the x-ray image and may contain information describing the examination carried out. For example, DICOM tags may be used to describe patient demographic data, information about the time and place the examination was carried out, physical parameters of the x-ray device used, and the type of examination carried out. DICOM tags enable the categorisation and identification of x-ray images of a patient. In some embodiments of the present invention, determining a set-up assessment value based on the patient pose and examination parameters of the x-ray device set-up, involves analysing information with respect to the view position of the x-ray imaging device, the identity of at least one anatomical feature of the patient, the laterality of the at least one anatomical feature. It may be understood by the skilled person that this information may be in a form compatible with the use of DICOM tags. In particular, the analysis of patient position data may involve at least one of: identifying at least one anatomical feature of the patient, determining the orientation and position of the at least one anatomical feature, determining the laterality of the at least one anatomical feature, and determining the view position of the x-ray imaging device. This information may then be incorporated into the x-ray image of the patient using DICOM tags.

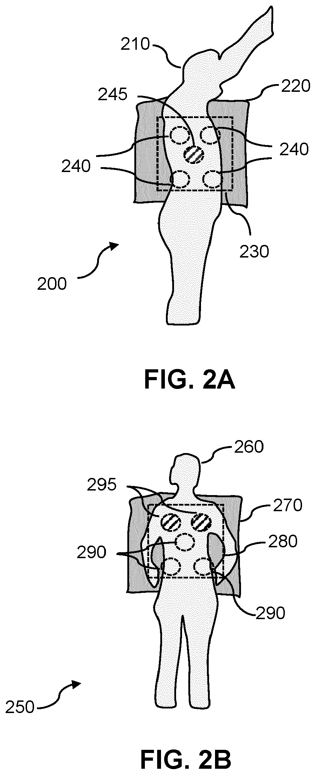

Referring now to Fig. 2A, there is depicted an illustration of a set-up 200 of a patient 210 and a medical x-ray imaging device 220 for a lateral chest examination of the patient, according to a proposed embodiment.

The area 230 outlines the field-of-view of the detector of the x-ray imaging device. The AEC chambers 240 form part of the x-ray imaging device. The x-ray imaging device 220 has been set-up for a lateral chest examination and therefore the central AEC chamber has been automatically activated. It is necessary that only the central chamber be activated as it is required that the chamber covers the location between the spine and the sternum.

AEC chambers comprise a device for automatic exposure termination in x-ray imaging devices. The chambers each measure the level of radiation in specific areas of the field-of-view throughout the examination procedure. When the radiation received by the chambers reaches a predetermined level, automatic termination of exposure occurs.

Referring now to Fig. 2B, there is depicted an illustration of a set-up 250 of a patient 260 and a medical x-ray imaging device 270 for a PA chest examination. The patient is positioned in the field-of view 280 of the x-ray imaging device. The AEC chambers 290 remain inactivated when the imaging device is in use, while the upper two chambers 295 are activated. This is to ensure the image covers the lungs without overlapping with the sternum.

In the case where the examination parameters of the x-ray imaging device do not correspond to the patient position, the incorrect AEC chambers may be selected. By way of example, if a lateral chest examination has been selected by the operator, but the patient is positioned for a PA chest examination, the central AEC chamber will be activated rather than the upper AEC chambers. The central AEC chamber overlaps the sternum and the spine and therefore this selection results in a reduced dose being detected by the chamber. This results in a delayed switch-off of the x-ray generator of the x-ray imaging device and therefore a higher patient dose.

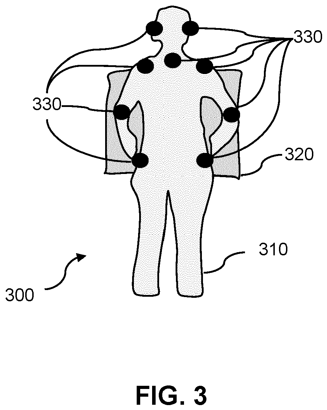

Referring now to Fig. 3, there is illustrated an RGB image 300 of a patient 310 and an x-ray imaging device 320, with key point identification used to determine a patient pose for a PA chest examination of a patient according to a proposed embodiment.

In this exemplary embodiment of the present invention, there is provided a video camera mounted at the collimator of the x-ray imaging device. This collects RGB data on the position of the patient to be examined. An illustration of such data is presented in Fig. 3, which depicts an RGB image 300 of a patient 310 and a medical x-ray imaging device 320. In some embodiments, the RGB image 300 may be analysed using a Convolution Neural Network (CNN) to identify a set of pre-selected anatomical key points 330. For each identified key point there is determined associated information about the type of anatomical feature, the laterality of the anatomical feature, and the orientation and position of the anatomical feature. Further, a probability is assigned to each of the key points, which describes the probability that the key point was correctly detected.

From the detected key points, the view position of the x-ray imaging device, the identity of at least one anatomical feature of the patient; and the laterality of at least one anatomical feature of the patient may be determined. This may be achieved in several ways. For example, to distinguish a posterior-anterior (PA) pose of the patient from an anterior-posterior (AP) pose the literalities of the shoulder key points may be compared. In a further example, a lateral pose may be distinguished from an AP/PA pose by measuring the distance between the shoulder key points.

In a further exemplary embodiment, three-dimensional position data (e.g., data from an RGB-D camera) may be used to increase the precision of the patient pose determination. In this example, a lateral pose may be distinguished from an AP/PA pose by analysing the depth distance between the shoulder key points.

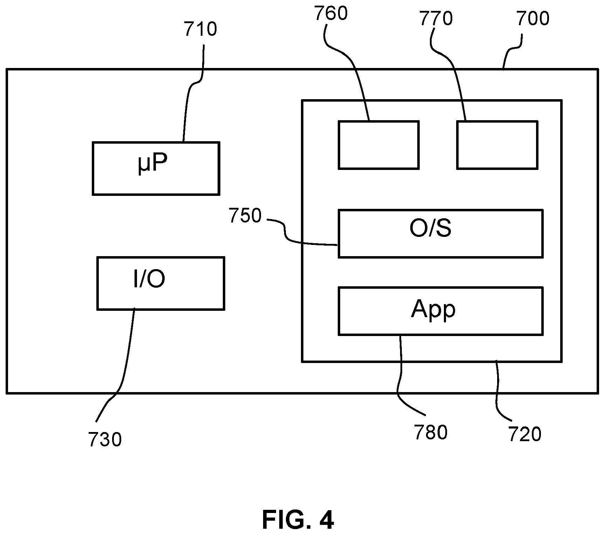

Fig. 4 illustrates an example of a computer 700 within which one or more parts of an embodiment may be employed. Various operations discussed above may utilize the capabilities of the computer 700. In this regard, it is to be understood that system functional blocks can run on a single computer or may be distributed over several computers and locations (e.g. connected via internet).

The computer 700 includes, but is not limited to, PCs, workstations, laptops, PDAs, palm devices, servers, storages, and the like. Generally, in terms of hardware architecture, the computer 700 may include one or more processors 710, memory 720 and one or more I/O devices 730 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 710 is a hardware device for executing software that can be stored in the memory 720. The processor 710 can be virtually any custom made or commercially available processor, a central processing unit (CPU), a digital signal processor (DSP), or an auxiliary processor among several processors associated with the computer 700, and the processor 710 may be a semiconductor based microprocessor (in the form of a microchip) or a microprocessor.

The memory 720 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 720 may incorporate electronic, magnetic, optical, and/or other types of storage media. Note that the memory 720 can have a distributed architecture, where various components are situated remote from one another, but can be accessed by the processor 710.

The software in the memory 720 may include one or more separate programs, each of which comprises an ordered listing of executable instructions for implementing logical functions. The software in the memory 720 includes a suitable operating system (O/S) 750, compiler 760, source code 770, and one or more applications 780 in accordance with exemplary embodiments. As illustrated, the application 780 comprises numerous functional components for implementing the features and operations of the exemplary embodiments. The application 780 of the computer 700 may represent various applications, computational units, logic, functional units, processes, operations, virtual entities, and/or modules in accordance with exemplary embodiments, but the application 780 is not meant to be a limitation.

The operating system 750 controls the execution of other computer programs, and provides scheduling, input-output control, file and data management, memory management, and communication control and related services. It is contemplated by the inventors that the application 780 for implementing exemplary embodiments may be applicable on all commercially available operating systems.

Application 780 may be a source program, executable program (object code), script, or any other entity comprising a set of instructions to be performed. When a source program, then the program is usually translated via a compiler (such as the compiler 760), assembler, interpreter, or the like, which may or may not be included within the memory 720, so as to operate properly in connection with the O/S 750. Furthermore, the application 780 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 730 may include input devices such as, for example but not limited to, a mouse, keyboard, scanner, microphone, camera, etc. Furthermore, the I/O devices 730 may also include output devices, for example but not limited to a printer, display, etc. Finally, the I/O devices 730 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 730 also include components for communicating over various networks, such as the Internet or intranet.

If the computer 700 is a PC, workstation, intelligent device or the like, the software in the memory 720 may further include a basic input output system (BIOS) (omitted for simplicity). The BIOS is a set of essential software routines that initialize and test hardware at start-up, start the O/S 750, and support the transfer of data among the hardware devices. The BIOS is stored in some type of read-only-memory, such as ROM, PROM, EPROM, EEPROM or the like, so that the BIOS can be executed when the computer 700 is activated.

When the computer 700 is in operation, the processor 710 is configured to execute software stored within the memory 720, to communicate data to and from the memory 720, and to generally control operations of the computer 700 pursuant to the software. The application 780 and the O/S 750 are read, in whole or in part, by the processor 710, perhaps buffered within the processor 710, and then executed.

When the application 780 is implemented in software it should be noted that the application 780 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 780 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The system of Fig. 1 may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A method for assessing the set-up of a patient and a medical x-ray imaging device for examination of the patient, wherein the method comprises:
analysing (110) patient position data from a position sensor to determine a pose of the patient to be examined, wherein the patient position data describes the position of the patient with respect to the x-ray imaging device; and
determining (120) a set-up assessment value based on the determined patient pose and at least one examination parameter of the x-ray device set-up.

2. The method of claim 1, wherein determining (120) a set-up assessment value based on the determined patient pose and the at least one examination parameter comprises: analysing information with respect to:
the view position of the x-ray imaging device;
the identity of at least one anatomical feature of the patient; and
the laterality of the at least one anatomical feature.

3. The method of claim 1, wherein determining (120) a set-up assessment value comprises:
determining (122), from the pose of the patient, expected examination parameters;
comparing (124) the expected examination parameters to the examination parameters of the x-ray device set-up; and optionally further comprising:
determining an alert signal based on the comparison of the expected examination parameters and the examination parameters of the x-ray device set-up.

4. The method of claim 1, wherein analysing (110) patient position data comprises:
identifying (112) at least one anatomical feature of the patient;
determining (114) the orientation and position of the at least one anatomical feature;
determining (116) the laterality of the at least one anatomical feature; and
determining the view position of the x-ray imaging device.

5. The method of claim 1, wherein analysing (110) patient position employs at least one neural network.

6. The method of claim 1, wherein the position sensor comprises at least one of:
an RGB camera;
an RGB-D camera;
a laser position sensor;
a radar position sensor;
an accelerometer;
a gyroscope; and
an ultrasound device.

7. The method of claim 1, wherein an examination parameter of the x-ray device set-up comprise at least one of:
an anatomical feature of the patient to be examined;
an orientation of an anatomical feature of the patient to be examined;
a laterality of an anatomical feature of the patient to be examined;
a view position of the x-ray imaging device; and
an automatic exposure control chamber of the x-ray imaging device to be used.

8. The method of claim 1, wherein a set-up assessment value comprises at least one of:
a value describing the suitability of the patient pose with respect to the examination parameters of the x-ray device set-up; and
a target adjustment to be made to the set-up.

9. The method of claim 8, wherein the target adjustment to be made to the set-up comprises at least one of:
an adjustment to the pose of the patient;
an adjustment to the position of the x-ray imaging device; and
an adjustment to the examination parameters of the x-ray device set-up.

10. A computer program comprising computer program code means adapted, when said computer program is run on a computer, to implement the method of any of claims 1-9.

11. A computer-readable medium having stored thereon the computer program product of claim 10.

12. A system for assessing the set-up of a patient and a medical x-ray imaging device for examination of the patient, comprising:
an analysis component (720) configured to analyse patient position data from a position sensor to determine a pose of the patient to be examined, wherein the patient position data describes the position of the patient with respect to the x-ray imaging device; and
a processor (710) configured to determine a set-up assessment value based on the determined patient pose and at least one examination parameter of the x-ray device set-up.

13. A medical x-ray imaging device comprising the system of claim 12, wherein determining a set-up assessment value on the determined patient pose and the at least one examination parameter, comprises analysing information with respect to:
the view position of the x-ray imaging device;
the identity of at least one anatomical feature of the patient; and
the laterality of the at least one anatomical feature.

14. A medical x-ray imaging device comprising the system of claim 12, wherein determining a set-up assessment value in the processor comprises:
determining, from the pose of the patient, expected examination parameters;
comparing the expected examination parameters to the examination parameters of the x-ray device set-up; and optionally further comprising:
determining an alert signal based on the comparison of the expected examination parameters and the examination parameters of the x-ray device set-up.

15. A medical x-ray imaging device comprising the system of claim 12, wherein a set-up assessment value comprises at least one of:
a value describing the suitability of the patient pose with respect to the examination parameters of the x-ray device set-up; and
a target adjustment to be made to the set-up.

16. A medical x-ray imaging device comprising the system of claim 12, wherein the target adjustment to be made to the set-up comprises at least one of:
an adjustment to the pose of the patient;
an adjustment to the position of the x-ray imaging device; and
an adjustment to the examination parameters of the x-ray device set-up.
